# EUROPÄISCHE PATENTSCHRIFT

(11) **EP 1 901 782 B1**
(45) Veröffentlichungstag und Bekanntmachung des Hinweises auf die Patenterteilung: **15.08.2012**
(21) Anmeldenummer: 06754128.4
(22) Anmeldetag: 06.06.2006
(51) Int. Cl.: A61K 49/12, A61K 49/14

(54) **VERFAHREN ZUM LÖSEN VON GASEN MIT KURZLEBIGEN PHYSIKALISCHEN EIGENSCHAFTEN IN EINER FLÜSSIGKEIT**
METHOD FOR DISSOLUTION OF GASES WITH SHORT-LIVED PHYSICAL PROPERTIES IN A LIQUID
PROCEDE DE DISSOLUTION DANS UN LIQUIDE DE GAZ AUX PROPRIETES PHYSIQUES EPHEMERES

(30) Priorität: 09.06.2005 DE 102005026604
(43) Veröffentlichungstag der Anmeldung: 26.03.2008
(73) Patentinhaber: Membrana GmbH, 42289 Wuppertal (DE); Max-Planck-Gesellschaft zur Förderung der Wissenschaften e.V., 80539 München (DE)
(72) Erfinder: BLÜMLER, Peter, 33613 Bielefeld (DE); LEMKE, Horst-Dieter, 63785 Obernburg (DE); KRIETER, Detlev, 97074 Würzburg (DE); SPIESS, Hans-Wolfgang, 55128 Mainz (DE); WIESE, Frank, 42389 Wuppertal (DE); ZÄNKER, Paul-Philipp, 55118 Mainz (DE)
(74) Vertreter: Schröder, Richard
(86) Internationale Anmeldenummer: PCT/EP2006/005349
(87) Internationale Veröffentlichungsnummer: WO 2006/131292

(56) Entgegenhaltungen:
- EP-A2- 0 620 447
- WO-A-95/27438
- WO-A-99/07415
- WO-A2-02/085417
- DE-A1- 2 833 493
- US-A1- 2002 094 317
- US-B1- 6 168 648

## Beschreibung

Die Erfindung betrifft ein Verfahren zum Lösen eines Gases mit kurzlebigen physikalischen Eigenschaften in einer Flüssigkeit.

Gase mit kurzlebigen physikalischen Eigenschaften sind z.B. radioaktive Gase oder hyperpolarisierte Gase. Radioaktive Gase sind Gase, die ein sog. Radionuklid bzw, ein Radioisotop enthalten. Solche Gase werden unter anderem in der Positronen-Emissions-Tomographie (PET) eingesetzt.

Die magnetische Resonanzspektroskopie (NMR) sowie bildgebende Verfahren auf Basis der magnetischen Resonanz, wie die Magnetresonanztomographie (MRT), verwenden neben anderen Substanzen hyperpolarisierte Gase als Kontrastmittel. Für bestimmte Anwendungen können unter Verwendung von hyperpolarisierten Gasen als Signalgeber verbesserte Bilder bzw. ein verbessertes Signal/Rauschverhältnis mit den genannten Verfahren auf Basis der magnetischen Resonanz erzielt werden.

Die Verwendung von hyperpolarisierten Edelgasen für NMR oder MRT Techniken ist z.B. beschrieben in US 6,491,895 B2, US 2001/0041834 A1 oder US 6,696,040 B2.

Das Problem bei der Verwendung von hyperpolarisierten Gasen in Lösung ist die rasche Depolarisation dieser Gase und die damit verbundenen geringen Halbwertszeiten, die im Bereich von einigen Sekunden liegen. Mit der Depolarisation geht aber gleichzeitig die Eigenschaft des Gases als verbesserter Signalgeber verloren.

Die US 6,488,910 B2 beschreibt eine Methode zum Einbringen von hyperpolarisiertem ³He und ¹²⁹Xe in eine Flüssigkeit mittels Mikroblasen. Die Flüssigkeit weist eine sehr geringe Löslichkeit für hyperpolarisiertes ³He und ¹²⁹Xe auf. Dabei soll ein Übergehen des hyperpolarisierten ³He und ¹²⁹Xe von der Mikroblase in die Flüssigkeit weitgehend verhindert oder zumindest gehemmt werden und so die Depolarisation minimiert und die Relaxationszeit verbessert werden.

WO 02l085417 offenbart ein Verfahren und eine Vorrichtung zum Anfeuchten von hyperpolarisierten Edelgases, welche als Signalgeber für Analyseverfahren, basierend auf magnetischer Resonanz, dienen.

US 6,284,222 offenbart eine Methode, hyperpolaisierten ³Xe in Gasform in Mikrovesikeln einzuschließen und ein Verfahren zu Herstellung einer injizierbaren Lösung oder Suspension enthaltend die hyperpolarisierten ³Xe beladenen Mikrovesikeln.

EP 0 968 000 B1 offenbart in den Blutkreislauf injizierbare Kontrastmittelformulierungen zur Verwendung für Analyseverfahren basierend auf magnetischer Resonanz bestehend aus Mikrovesikeln, die mit einer Mischung aus hyperpolarisierten Edelgas und einem Inertgas mit hohem Molekulargewicht gefüllt sind. Die Gegenwart des Inertgases soll das hyperpolarisierte Edelgas dahingehend stabilisieren, dass ein Verlassen des hyperpolarisierten Gases durch die Vesikelhülle vermieden wird und das hyperpolarisierte Gas in den Mikrovesikeln verbleibt.

Nach dem Stand der Technik wird mit relativ aufwändigen Methoden versucht, Gase mit kurzlebigen physikalischen Eigenschaften, insbesondere hyperpolarisierte Gase, so in eine Flüssigkeit zu bringen, dass das Gas weiterhin in der Gasphase vorliegt, um eine Verringerung der Halbwertszeit durch Lösen des Gases der Flüssigkeit zu minimieren.

Aufgabe der vorliegenden Erfindung ist es, ein Verfahren zur Verfügung zu stellen, mittels dessen Gase mit kurzlebigen physikalischen Eigenschaften auf einfache und gleichzeitig effiziente Weise in Lösung gebracht werden können.

Diese Aufgabe wird gelöst durch ein Verfahren zum Lösen eines Gases mit kurzlebigen physikalischen Eigenschaften in einer Flüssigkeit, enthaltend die Schritte, Bereitstellung des Gases mit kurzlebigen physikalischen Eigenschaften, Bereitstellung der Flüssigkeit, Einbringen des Gases mit kurzlebigen physikalischen Eigenschaften in die Flüssigkeit, wobei das Verfahren dadurch gekennzeichnet ist, dass das Gas mit kurzlebigen physikalischen Eigenschaften ein hyperpolarisiertes Gas ist und das Gas, über eine semipermeable, gasdurchlässige Membran der Flüssigkeit zugeführt wird, wobei die Membran so in einem Gehäuse eingebettet ist, dass das Gehaüse durch die Membran in mindestens einem Raum für die Flussigkeit und mindestens einem Raum für das Gas geteilt wird, und der Raum für die Flüssigkeit mindestens einen Einlass und einen Auslass aufweist und der Raum für das Gas mindestens einen Einlass für das Gas aufweist, über den das Gas an die Membran herangeführt wird.

Völlig überraschend hat sich gezeigt, dass das erfindungsgemäße Verfahren mit semipermeablen, gasdurchlässigen Membranen die Halbwertszeit der Gase mit kurzlebigen physikalischen Eigenschaften in deutlich geringerem Maße beeinflusst als ein Verfahren zur direkten Lösung der Gase in der Flüssigkeit. Die Halbwertszeit des Gases mit kurzlebigen physikalischen Eigenschaften wird mit dem erfindungsgemäßen Verfahren auf hohem Niveau belassen. Das gilt insbesondere für hyperpolarisierte Gase, da sich gezeigt hat, dass semipermeable, gasdurchlässige Membranen die hyperpolarisierten Gase nur wenig depolarisieren.

Durch die Verwendung von Membranen lässt sich gleichzeitig eine direkte molekulare Lösung der Gase mit kurzlebigen physikalischen Eigenschaften in der Flüssigkeit erreichen. Für den Einsatz solcher Lösungen ist es sehr oft wichtig, dass man eine stabile Lösung der Gase erreicht. Gasblasen -auch mikroskopisch kleine- und Einschlussverbindungen sind entweder in einem instabilen Zustand oder halten die Gase in Molekülen eingeschlossen. Bei in Molekülen eingeschlossenen Gasen ist die Relaxationszeit zwar relativ lang, die freie Diffusivität geht jedoch verloren, was die Verwendung als Kontrastmittel stark eingeschränkt. Darüber hinaus beinhaltet die Anwendung der mit Gas angereicherten Flüssigkeit als Kontrastmittel unter Einsatz instabiler Lösungen die Gefahr der spontanen Bildung größerer Gasblasen und damit die Gefahr von Gefäßverschlüssen und Embolien, woraus deutliche Einschränkungen in der Anwendung resultieren. Zusätzlich wird durch die molekulare Lösung die Schaumbildung umgangen. Die Lösungen sind schnell und sicher herstellbar und leicht zu handhaben. Mit Membranen lassen sich kontinuierlich und deshalb pro Zeiteinheit höhere Gasmengen in Flüssigkeit lösen, als das durch die Einbringung von Gasblasen in Flüssigkeit möglich ist. Weiterhin ist darauf zu achten, dass alle Oberflächen, die mit dem Gas bzw. der Flüssigkeit in Kontakt sind, aus möglichst sauberen, inerten Materialien mit geringen Unterschieden in ihrer magnetischen Suszeptibilität bestehen.

Als Gase mit kurzlebigen physikalischen Eigenschaften sind im Rahmen der vorliegenden Erfindung solche zu verstehen, deren spezifische physikalische Eigenschaft mit der Zeit abklingt und zwar mit Halbwertszeiten zwischen einigen Millisekunden und mehreren Stunden.

Dazu gehören, wie eingangs bereits erwähnt, radioaktive Gase oder hyperpolarisierte Gase. Radioaktive Gase werden z.B. in der Positronen-Emissions-Tomographie (PET) eingesetzt. Die radioaktiven Gase enthalten sog. Radioisotope bzw. Radionuklide.

Hyperpolarisierte Gase werden für die magnetische Resonanzspektroskopie (NMR) oder die Kernspintomographie bzw. Magnetresonanztomographie (MRT) eingesetzt. Durch den Einsatz hyperpolarisierter Gase wird das Signal/Rauschverhältnis deutlich verbessert. Zum Einsatz kommen hier vor allem die Xenon lsotope ¹²⁹Xe bzw. ¹³¹Xe oder das Heliumisotop ³He.

Darüber hinaus sind noch folgende lsotope für Gasverbindungen als relevant zu nennen:

| Isotop | Halbwertszeit | Gasförmige Verbindung (Bsp.) | Isotop | Halbwertszeit | Gasförmige verbindung (Bsp.) |
|---|---|---|---|---|---|
| ¹⁰C | 19.5 s | CO₂, CH₄, | ⁸⁸Kr | 2.8 h | Kr |
| ¹¹C | 20 min | FC/KW* | ⁸⁹Kr | 3.2 min | |
| ¹⁵C | 2.5s | | ⁹⁰Kr | 33 s | |
| ¹³N | 10min | N₂, N₂O, NH₃ | ⁹¹Kr | 10 s | |
| ¹⁶N | 7 s | | ⁹²Kr | 3 s | |
| ¹⁷N | 4.2 s | | ⁹³Kr | 2 s | |
| ¹⁴O | 70s | O₂, CO₂ | ⁹⁴Kr | 1.4 s | |
| ¹⁵O | 123 s | | ¹¹⁸Xe | 6 min | Xe |
| ¹⁹O | 29 s | | ¹¹⁹Xe | 6 min | |
| ²⁰O | 14 s | | ¹²⁰Xe | 40 min | |
| ¹⁷F | 66 s | SF₆, FCKW* | ¹²¹Xe | 40 min | |
| ¹⁸F | 109 min | | ¹²²Xe | 20 h | |
| ²⁰F | 11 s | | ¹²³Xe | 2h | |
| ²¹F | 4.3 s | | ¹²⁵Xe | 17h | |
| ²²F | 4 s | | ^{125m}Xe | 60 s | |
| ¹⁸Ne | 1.5 s | Ne | ^{127m}Xe | 75 s | |
| ¹⁹Ne | 17 s | | ¹³⁵Xe | 9.2 h | |
| ²³Ne | 37 s | | ^{135m}Xe | 15.6 min | |
| ²⁴Ne | 3.4 min | | ¹³⁷Xe | 4 min | |
| ³⁰S | 1.4 s | SF₆ | ¹³⁶Xe | 17.5 min | |
| ³¹S | 2.7 s | | ¹³⁹Xe | 43 s | |
| ³⁷S | 5 min | | ¹⁴⁰Xe | 16 s | |
| ³⁸S | 2.9 h | | ¹⁴¹Xe | 1.7 s | |
| ³³Cl | 2.5 s | FCKW* | ²⁰²Rn | 13 s | Rn |
| ³⁴Cl | 1.6 s | | ²⁰³Rn | 45 s | |
| ^{34m}Cl | 32 min | | ²⁰³mRn | 28 s | |
| ³⁸Cl | 37.3 min | | ²⁰⁴Rn | 75 s | |
| ³⁹Cl | 55 min | | ²⁰⁵Rn | 1.8 min | |
| ⁴⁰Cl | 1.4 min | | ²⁰⁶Rn | 6.5 min | |
| ³⁵Ar | 1.8 s | Ar | ²⁰⁷Rn | 11 min | |
| ⁴¹Ar | 1.8 h | | ²⁰⁸Rn | 23 min | |
| ⁷⁴Kr | 20 min | Kr | ²⁰⁹Rn | 30 min | |
| ⁷⁵Kr | 5.5 min | | ²¹⁰Rn | 2.5 h | |
| ⁷⁶Kr | 14.8 h | | ²¹¹Rn | 15 h | |
| ⁷⁷Kr | 1.2 h | | ²¹²Rn | 25 min | |
| ^{79m}Kr | 55 s | | ²¹⁹Rn | 4 S | |
| ^{81m}Kr | 13 s | | ²²⁰Rn | 55 s | |
| ^{83m}Kr | 1.9 h | | ²²¹Rn | 25 min | |
| ^{85m}Kr | 4.4 h | | ²²³Rn | 43 min | |
| ⁸⁷Kr | 76 min | | ²²⁴Rn | 1.9 h | |

Weiterhin sind auch molekulare Gase durch Hydrierung geeigneter Moleküle mit Doppel- bzw. Dreifachbindungen mit para-¹H₂ oder ortho-²H₂ (sog. PHIP bzw. PASADENA-Experiment) zugänglich. Die Polarisation der Wasserstoffisotope lässt sich dabei im Tieffeld (ALTADENA-Effekt) auch auf andere Isotope mit einem Kernspin übertragen (z.B. ¹³C oder ¹⁹F).

In einer bevorzugten Ausführungsform ist das erfindungsgemäße Verfahren dadurch gekennzeichnet, dass das Gas mit kurzlebigen physikalischen Eigenschaften blasenfrei über die Membran der Flüssigkeit zugeführt wird. Dadurch wird eine molekulare Lösung der Gase mit kurzlebigen physikalischen Eigenschaften erhalten, ohne dass eine Schaumbildung in der Lösung auftritt. Das Verfahren ist daher für die verschiedensten Flüssigkeiten, Lösungen und Emulsionen (amphiphile Substanzen, Proteinlösungen, Blutplasma, Fettemulsionen, etc.), die teilweise zu starker Schaumbildung neigen, geeignet. Die Flüssigkeiten sollten dabei möglichst frei von para- und ferromagnetischen Verunreinigungen sein, da diese zu rascher Depolarisation hyperpolarisierter Gase führen würden. Auf die Entfernung von gelöstem O₂ ist deshalb zu achten.

In der Ausführungsform des erfindungsgemäßen Verfahrens ist die Membran so in einem Gehäuse eingebettet, dass das Gehäuse durch die Membran in mindestens einen Raum für die Flüssigkeit und mindestens einen Raum für das Gas geteilt wird und dass der Raum für die Flüssigkeit mindestens einen Einlass und einen Auslass aufweist und der Raum für das Gas mindestens einen Einlass für das Gas aufweist, über den das Gas an die Membran herangeführt wird. Besonders bevorzugt ist ein kontinuierlicher Gegenstrom von Gas und Flüssigkeit, durch den das Gas in der Lösung angereichert wird.

Bei der im erfindungsgemäßen Verfahren eingesetzten Membran kann es sich um eine Flachmembran oder um mindestens eine Hohlfasermembran handeln. Vorzugsweise werden Hohlfasermembranen eingesetzt, wobei die Hohlfasermembranen zu einem Bündel angeordnet sind.

Besonders vorteilhaft sind solche Hohlfasermembranen, die einen Außendurchmesser zwischen 30 und 3000µm, bevorzugt zwischen 50 und 500µm aufweisen. Günstig ist eine Wanddicke der Hohlfasermembran zwischen 5 und 150µm, besonders günstig eine Dicke zwischen 10 und 100µm.

Die im erfindungsgemäßen Verfahren eingesetzte Membran muss auf der einen Seite eine hinreichende Durchlässigkeit für das Gas mit kurzlebigen physikalischen Eigenschaften aufweisen und auf der anderen Seite über einen ausreichenden Zeitraum undurchlässig gegenüber der Flüssigkeit, der das Gas zugeführt wird, sein.

Die erfindungsgemäß verwendete Membran kann hydrophil oder hydrophob sein. Für den Fall, dass eine hydrophile Membran eingesetzt wird, weist diese bevorzugt eine gegenüber einem Flüssigkeitsdurchtritt dichte aber gasdurchlässige Trennschicht auf. Vorzugsweise handelt es sich bei der erfindungsgemäß eingesetzten Membran aber um eine hydrophobe Membran. In einer vorteilhaften Ausgestaltung weist diese hydrophobe Membran eine durchgehend mikroporöse Struktur über ihre Wand auf. Derartige Membranen werden beispielsweise in der DE 28 33 493 C3, DE 32 05 289 C2 oder GB 2 051 665 beschrieben.

In einer weiteren vorteilhaften Ausgestaltung weist die hydrophobe Membran eine integral asymmetrische Struktur auf mit einer mikroporösen Stützschicht und einer an diese Stützschicht angrenzenden dichten oder nanoporösen Trennschicht. Solche Membranen mit dichter oder nanoporöser Trennschicht werden z.B. in EP 299 381 A1, WO 99/04891 oder WO 00/43114 beschrieben.

Die Membranen zum Lösen von Gas mit kurzlebigen physikalischen Eigenschaften in einer Flüssigkeit bestehen vorzugsweise aus einem Polymer auf der Basis von Polyolefinen. Hierbei kann es sich um ein einzelnes Polyolefin oder um eine Mischung aus mehreren Polyolefinen handeln.

Besonders bevorzugt zum Lösen von Gas mit kurzlebigen physikalischen Eigenschaften in einer Flüssigkeit sind solche Membranen, die aus Polypropylen, Polyethylen, Polymethylpenten sowie darauf basierenden Modifikationen,

Copolymeren oder Blends oder Mischungen dieser Polyolefine untereinander oder mit anderen Polyolefinen gefertigt sind.

Zudem sind Membranen, die aus Silikonverbindungen bestehen oder eine Silikonbeschichtung aufweisen, für das erfindungsgemäße Verfahren geeignet. Darüber hinaus sind noch Membranen, die aus einem Polymer auf der Basis eines aromatischen Sulfonpolymers, wie z.B. Polysulfon, Polyethersulfon oder Polyarylethersulfon, als geeignet zu nennen.

Das Verfahren zum Lösen von Gas mit kurzlebigen physikalischen Eigenschaften in einer Flüssigkeit ist besonders geeignet zum Lösen von hyperpolarisierten Gasen.

Bevorzugt ist das erfindungsgemäße Verfahren dadurch gekennzeichnet, dass das hyperpolarisierte Gas Xenon ist.

Es können die verschiedensten Flüssigkeiten zum Lösen von Gas mit kurzlebigen physikalischen Eigenschaften verwendet werden. Als Beispiel wären neben den bereits genannten Flüssigkeiten Lipofundin, Blutplasma, Glukoselösung oder halogenierte Blutersatzstoffe (Perfluorcarbone) zu nennen. Bevorzugt ist die Flüssigkeit, in der das hyperpolarisierte Gas gelöst wird, eine physiologische Flüssigkeit.

In einem besonders bevorzugten Verfahren ist die Flüssigkeit, in der das hyperpolarisierte Gas gelöst wird, Blut.

Eine Lösung von hyperpolarisiertem Gas in einer Flüssigkeit, hergestellt nach dem erfindungsgemäßen Verfahren, ist als Kontrastmittel für analytische Methoden, basierend auf magnetischer Resonanz, zu verwenden. Der Nutzen hyperpolarisierter Gase als Kontrastmittel für analytische Methoden, basierend auf magnetischer Resonanz, ist ausführlich in US Patent 6,630,126 B2 beschrieben.

Eine Lösung von hyperpolarisiertem Gas in einer Flüssigkeit, hergestellt nach dem erfindungsgemäßen Verfahren, kann ebenfalls eingesetzt werden für:
● Xe-NMR bzw. SPINOE-NMR im Bereich der Biotechnologie zur Aufklärung der Struktur von Riesenmolekülen bzw. der Dynamik von Proteinen
● Oberflächen sensitive Xe-SPINOE oder CP-NMR [9] in biologischen Membranen oder von komplexen Biomolekülen an dünnen Schichten.
● Hoch- und Niederfeld-MRT und funktionelle MRT an Lebewesen

Besonders bei biophysikalischen Untersuchungen, für die häufig Wasser als Lösungsmittel verwendet wird, konnten bislang nur unzureichende Konzentrationen von hyperpolarisiertem Xenon in Wasser realisiert werden. Mit dem erfindungsgemäßen Verfahren ist es möglich, die Konzentration von hyperpolarisiertem Xenon in Wasser oder wässrigen Lösungen so stark zu erhöhen, dass es auch für Untersuchungen in Wasser als Kontrastmittel eingesetzt werden kann. Dies wird dadurch erreicht, dass das hyperpolarisierte Xenon unter erhöhtem Druck vorzugsweise durch Hohlfasermembranen geleitet wird. Vorteilhafterweise wird das hyperpolarisierte Xenon dabei auch blasenfrei gelöst.

Das erfindungsgemäße Verfahren zur Lösung von hyperpolarisiertem Gas in einer Flüssigkeit ist sowohl für bildgebende Analyseverfahren wie Kernspintomographie bzw. Magnetresonanztomographie (MRT) als auch für spektroskopische Analyseverfahren auf basierend auf magnetischer Resonanz wie die magnetische Resonanzspektroskopie (NMR) geeignet.

Besonders bevorzugt ist die Verwendung einer Lösung von hyperpolarisiertem Gas in einer Flüssigkeit, hergestellt nach dem erfindungsgemäßen Verfahren, als Kontrastmittel für analytische Methoden basierend auf magnetischer Resonanz zum Einsatz für menschliche oder tierische Organe und Gewebe.

Die Erfindung wird anhand der nachfolgenden Beispiele näher erläutert, jedoch wird der Bereich der Erfindung dadurch nicht eingeschränkt.

Zur Durchführung des erfindungsgemäßen Verfahrens wurden in den nachfolgend beschriebenen Beispielen 1 und 2 zwei Anordnungen verwendet, mit denen hyperpolarisiertes Xenon kontinuierlich in ein Lösungsmittel eingebracht wurde. Die Anordnungen unterscheiden sich durch die Art der Flüssigkeitspumpe. Bei Anordnung 1 wird die Flüssigkeit manuell mit zwei Spritzen durch den die Hohlfasermembran umgebenden Außenraum eines Hohlfasermembranmoduls und ein damit verbundenes Reservoir hin- und hergepumpt. Der Hohlfasermembranmodul enthielt mikroporöse Polypropylenmembranen, wie sie üblicherweise zur Oxygenation von Blut eingesetzt werden (Oxyphan PP50/280, Firma Membrana GmbH). Das hyperpolarisierte Xenon wurde durch die Lumina der Hohlfasermembranen des Membranmoduls gepumpt und diffundierte dabei teilweise in die Flüssigkeit hinein. Von hier aus wurde das gelöste Gas in ein Reservoir weitertransportiert, wo es durch eine NMR-Messung detektiert werden konnte. Um die Effektivität der Methode für verschiedene Lösungsmittel vergleichen zu können, wurde neben dem Reservoir auch ein Schlauch mit dem aus dem Membranmodul austretenden Xenongas durch die NMR-Spule geführt. Die NMR-Spektren können so auf das Signal des gasförmigen Xenons referenziert werden.

Bei der zweiten Anordnung wurde die Flüssigkeit mit einer Druckluftmembranpumpe zirkulierend durch den Versuchsaufbau gepumpt. Da die verwendete Pumpe keinerlei metallische oder magnetische Teile enthält, kann sie auch in starken Magnetfeldern betrieben werden. Somit konnte mit diesem Prototyp kontinuierlich hyperpolarisierte Xenonlösung hergestellt werden. Der Gasvolumenstrom betrug in beiden Anordnungen ca. 200ml/min bei einem Gasüberdruck von etwa 100mbar. Der Flüssigkeitsvolumenstrom wurde auf ca. 240 ml/min eingestellt.

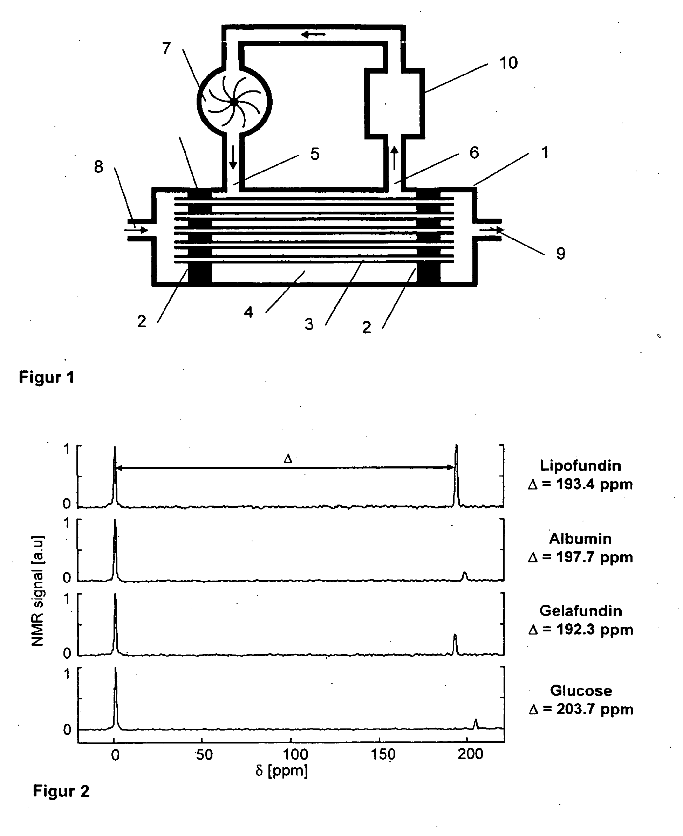

Figur 1 stellt die zweite Anordnung zum Lösen von hyperpolarisertem Xenon in einer Flüssigkeit schematisch dar. Die Anordnung besteht aus einem Hohlfasermodul 1 mit an ihren Enden in Vergussmassen 2 eingebetteten Hohlfasern 3. Der Außenraum 4 um die Hohlfasern 3 wird über Eingangsanschluss 5 und Ausgangsanschluss 6 mit der Flüssigkeit durchströmt. In der in Figur 1 dargestellten Anordnung wird die Flüssigkeit über eine Pumpe 7 umgepumpt. Alternativ kann die Flüssigkeit, wie bezüglich Anordnung 1 beschrieben, auch manuell hin- und her bewegt werden. Lumenseitig werden die Hohlfasern 3 über Einlassanschluss 8 und Auslassanschluss 9 mit hyperpolarisiertem Xenon beaufschlagt. Das in der Flüssigkeit gelöste hyperpolarisierte Xenon wird in der NMR Messzelle 10 detektiert.

### Beispiel 1:

Mittels Anordnung 1 wurden verschiedene Versuche zur Einbringung hyperpolarisierten Xenons in verschiedenen medizinisch/biologisch relevanten Flüssigkeiten durchgeführt. Die verwendeten Lösungsmittel sind medizinisch unbedenklich und befinden sich bereits in klinischem Einsatz. Lipofundin 20% ist eine fetthaltige, Albumin eine eiweisshaltige Nährlösung für Komapatienten. Gelafundin wird als Plasmaexpander bei hohem Blutverlust eingesetzt. Glucoselösungen wären vor allem als Kontrastmittel zur Bildgebung am Gehirn interessant. In allen Versuchen konnte das hyperpolarisierte Xenon über die Membran ausschließlich in molekularer Form in der jeweiligen Flüssigkeit gelöst werden. In keiner Lösung war Xenon als Gas, d.h. in nicht gelöster Form, nachweisbar. Figur 2 stellt die ¹²⁹Xe NMR-Spektren in den verschiedenen Lösungsmitteln graphisch dar. Um die gemessenen und in Figur 2 dargestellten NMR-Spektren auf das Signal des gasförmigen hyperpolarisierten Xenons zu referenzieren (chemische Verschiebung von 0 ppm), wurde ein weiterer Schlauch mit gasförmigem hyperpolarisiertem Xenon als externer Standard durch das Messvolumen geführt. Das NMR-Signal des gelösten Xenons konnte in jeder Flüssigkeit bei einer für jede Flüssigkeit charakteristischen chemischen Verschiebung Δ zwischen 192 und 204 ppm gemessen werden. Folglich ist es möglich, Xenon ohne Verlust der Spinpolarisation, d.h. ohne messbare Depolarisation durch die Membran in die verschiedenen Lösungsmittel einzubringen. Die unterschiedlichen Intensitäten der Xenonsignale im gelösten Zustand lassen sich durch die unterschiedlichen Konzentrationen der Substanzen und der individuellen Lösbarkeit von Xenon in diesen Substanzen erklären.

### Beispiel 2:

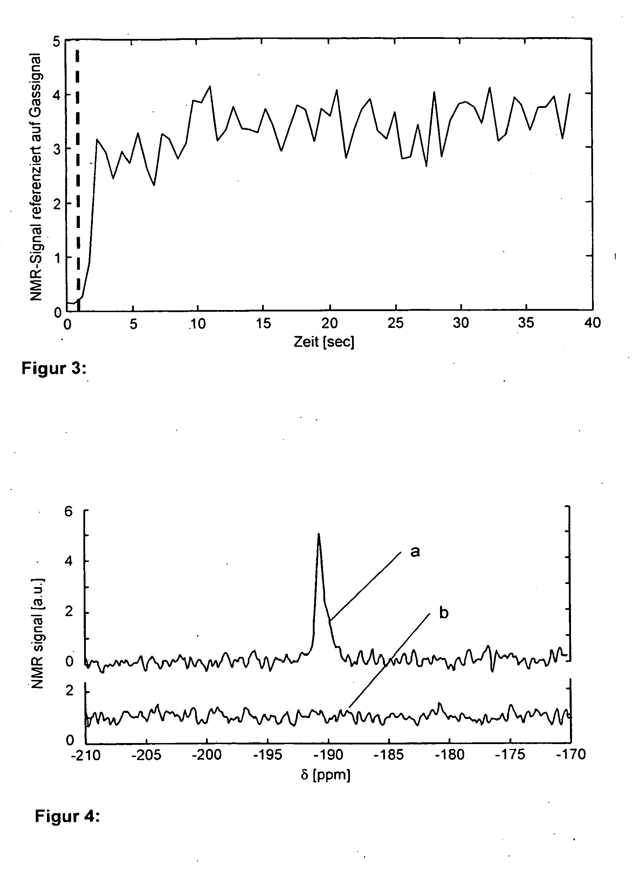

Mittels Anordnung 2 wurde hyperpolarisiertes Xenon über einen Hohlfasermembranmodul in Lipofundin eingebracht. Figur 3 zeigt die Signalstärke des in Lipofundin gelösten hyperpolarisierten Xenons in Abhängigkeit von der nach dem Einschalten der Pumpe vergangenen Zeit. Die unterbrochene Linie in Figur 3 markiert den Einschaltzeitpunkt der Pumpe. Die zweite Linie markiert die ermittelte Signalkurve des in Lipofundin gelösten hyperpolarisierten Xenons. Ein deutlicher Signalanstieg auf etwa 75% des Maximalsignals ist bereits nach 2 Sekunden zu erkennen. Der Maximalwert wird nach einer Zeit von etwa 10 Sekunden erreicht. Damit wird deutlich, dass über semipermeable, gasdurchlässige Membranen in Lösung gebrachtes hyperpolarisiertes Xenon praktisch sofort in der Lösung zu Verfügung steht.

### Beispiele 3 und 4, Vergleichsbeispiele 3a und 4a:

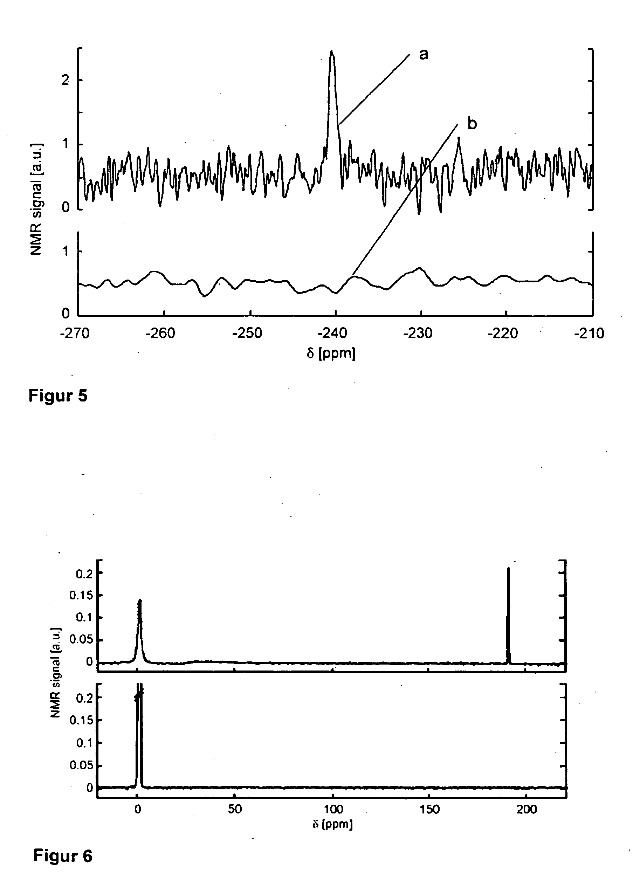

In den Beispielen 3 und 4 wurde ¹²⁹Xe über eine Membran in analoger Weise zu Beispiel 2 in Lipofundin (Beispiel 3) bzw. DMSO (Beispiel 4) eingebracht. Zum Vergleich wurden diese Flüssigkeiten mit ¹²⁹Xe überschichtet und von ¹²⁹Xe überströmt. Einfaches Überschichten oder Überströmen der Flüssigkeiten mit Gas lieferte weder bei der Flüssigkeit Lipofundin (Vergleichsbeispiel 3a) noch bei DMSO (Vergleichsbeispiel 4a) ein messbares Signal, wohingegen das beschriebene erfindungsgemäße membranvermittelte Verfahren in beiden Fällen erfolgreich war. Beispiel 3 und Vergleichsbeispiel 3a sind in Figur 4 dargestellt. Die obere Signalkurve a zeigt das ¹²⁹Xe Signal in Lipofundin für Beispiel 3 gemäß des Verfahrens der vorliegenden Erfindung und die untere Signalkurve b zeigt das ¹²⁹Xe Signal in Lipofundin für Vergleichsbeispiel 3a, ermittelt unter Anwendung der Überschichtungsmethode. Beispiel 4 und Vergleichsbeispiel 4a sind in Figur 5 dargestellt. Figur 5 zeigt einen Vergleich des ¹²⁹Xe Signals gelöst in DMSO, wobei die obere Signalkurve a (Beispiel 4) für das Verfahren gemäß der vorliegenden Erfindung und die untere Signalkurve b (Vergleichsbeispiel 4a) für die Überschichtungsmethode ermittelt wurde. Die Signalkurve a zeigt in beiden Fällen einen für gelöstes hyperpolarisiertes ¹²⁹Xe charakteristischen Peak, der in den Signalkurven b nicht festgestellt werden konnte.

### Beispiel 5, Vergleichsbeispiel 5a:

Hyperpolarisiertes Xenon wurde unter Verwendung eines Hochdruck-Hohlfasermoduls in Wasser eingebracht. Der Gasdruck des durch die Hohlfasern strömenden hyperpolarisierten Xenons betrug 7 bar. Das Hochdruck-Hohlfasermodul enthielt Hohlfasermembranen des Typs X50, Firma Celgard. Diese hydrophoben Membranen auf der Basis von Polypropylen werden typischerweise für die Be- und Entgasung von Flüssigkeiten eingesetzt. Es konnte gezeigt werden, dass hyperpolarisiertes Xenon mit dem erfindungsgemäßen Verfahren in vergleichsweise großen Mengen in Wasser gelöst werden kann, obwohl der Löslichkeitskoeffizient von Xenon in Wasser einen sehr geringen Wert aufweist. Figur 6 zeigt NMR Spektren von hyperpolarisiertem Xenon in Wasser, aufgenommen unter Anwendung des erfindungsgemäßen Verfahrens bei erhöhtem Gasdruck von 7 bar (Beispiel 5), und von hyperpolarisiertem Xenon in Wasser aufgenommen unter Anwendung der Überschichtungsmethode ebenfalls bei erhöhtem Gasdruck von 7 bar (Vergleichsbeispiel 5a). Das zur Kontrolle ermittelte Signal des eingesetzten gasförmigen hyperpolarisierten Xenons liegt bei 0 ppm, das Signal des in Wasser gelösten hyperpolarisierten Xenons liegt bei etwa 190 ppm. Die obere Signalkurve (Beispiel 5) wurde unter Anwendung des erfindungsgemäßen Verfahrens ermittelt. In dieser Signalkurve ist ein starkes Signal von gelöstem hyperpolarisierten Xenon bei 190 ppm zu erkennen. Die untere Kurve (Vergleichsbeispiel 5a) wurde unter Anwendung der Überschichtungsmethode ermittelt. Hier ist kein gelöstes hyperpolarisiertes Xenon detektierbar. Anhand des Beispiels 5 und des Vergleichsbeispiels 5a konnte gezeigt werden, dass die Effizienz des erfindungsgemäßen Verfahrens zum Lösen eines Gases mit kurzlebigen physikalischen Eigenschaften in einer Flüssigkeit durch Erhöhung des Gasdrucks weiter gesteigert werden kann. Hierdurch lassen sich amphiphile Substanzen in wässriger Lösung gut mit Xenon beschicken.

## Patentansprüche

1. Verfahren zum Lösen eines Gases mit kurzlebigen physikalischen Eigenschaften in einer Flüssigkeit umfassend die Schritte, Bereitstellung des Gases mit kurzlebigen physikalischen Eigenschaften, Bereitstellung der Flüssigkeit, Einbringen des Gases mit kurzlebigen physikalischen Eigenschaften in die Flüssigkeit, **dadurch gekennzeichnet, dass** das Gas mit kurzlebigen physikalischen Eigenschaften ein hyperpolarisiertes Gas ist und das Gas über eine semipermeable, gasdurchlässige Membran der Flüssigkeit zugeführt wird, wobei die Membran so in einem Gehäuse eingebettet ist, dass das Gehäuse durch die Membran in mindestens einen Raum für die Flüssigkeit und mindestens einen Raum für das Gas geteilt wird, und der Raum für die Flüssigkeit mindestens einen Einlass und einen Auslass aufweist und der Raum für das Gas mindestens einen Einlass für das Gas aufweist, über den das Gas an die Membran herangeführt wird.

2. Verfahren nach Anspruch 1 **dadurch gekennzeichnet, dass** das Gas blasenfrei in der Flüssigkeit gelöst wird.

3. Verfahren nach einem oder mehreren der Ansprüche 1 bis 2, **dadurch gekennzeichnet, dass** die Membran mindestens eine Hohlfasermembran ist.

4. Verfahren nach einem oder mehreren der Ansprüche 1 bis 3, **dadurch gekennzeichnet, dass** die Membran hydrophob ist.

5. Verfahren nach Anspruch 4, **dadurch gekennzeichnet, dass** die Membran eine mikroporöse Struktur aufweist.

6. Verfahren nach Anspruch 5, **dadurch gekennzeichnet, dass** die Membran eine dichte oder nanoporöse Trennschicht aufweist.

7. Verfahren nach einem oder mehreren der Ansprüche 1 bis 6, **dadurch gekennzeichnet, dass** die Membran aus einem Polymer auf der Basis mindestens eines Polyolefins besteht.

8. Verfahren nach Anspruch 7, **dadurch gekennzeichnet, dass** das mindestens eine Polyolefin Polypropylen oder Polymethylpenten ist.

9. Verfahren nach einem oder mehreren der Ansprüche 1 bis 3, **dadurch gekennzeichnet, dass** die Membran eine Silikonmembran ist oder eine Silikonbeschichtung aufweist.

10. Verfahren nach einem oder mehreren der Ansprüche 1 bis 6, **dadurch gekennzeichnet, dass** die Membran aus einem Polymer auf der Basis eines aromatischen Sulfonpolymers besteht.

11. Verfahren nach einem oder mehreren der Ansprüche 1 bis 10. **dadurch gekennzeichnet, dass** das hyperpolarisierte Gas Xenon ist.

12. Verfahren nach einem oder mehreren der Ansprüche 1 bis 11, **dadurch gekennzeichnet, dass** die Flüssigkeit, in der das hyperpolarisierte Gas gelöst wird, eine physiologische Flüssigkeit ist.

13. Verfahren nach Anspruch 12, **dadurch gekennzeichnet, dass** die physiologische Flüssigkeit Blutplasma ist.

14. Verfahren nach Anspruch 12, **dadurch gekennzeichnet, dass** die physiologische Flüssigkeit Blut ist.

15. Verwerdung einer Lösung von hyperpolarisiertem Gas in einer Flüssigkeit, hergestellt nach einem Verfahren gemäß einem oder mehreren der Ansprüche 1 bis 14, als Kontrastmittel für analytische Methoden basierend auf magnetischer Resonanz.

16. Verwendung einer Lösung von hyperpolarisiertem Gas in einer Flüssigkeit, hergestellt nach einem Verfahren gemäß einem oder mehreren der Ansprüche 1 bis 14, als Kontrastmittel für analytische Methoden basierend auf magnetischer Resonanz zum Einsatz für menschliche oder tierische Organe und Gewebe.

## Claims

1. Process for dissolving a gas with short-lived physical properties in a liquid, comprising the steps of providing the gas with short-lived physical properties, providing the liquid and bringing the gas with short-lived physical properties into the liquid, said process being **characterised in that** the gas with short-lived physical properties is a hyperpolarised gas and that the gas is fed into the liquid via a semi-permeable membrane that is permeable to gas, said membrane being embedded in a housing in such a way that the housing is split by the membrane into at least one space for the liquid and at least one space for the gas, and that the space for the liquid has at least one inlet and one outlet and the space for the gas has at least one inlet for the gas via which the gas is admitted to the membrane.

2. Process according to Claim 1, **characterised in that** the gas is dissolved in the liquid bubble-free.

3. Process according to one or more of Claims 1 to 2, **characterised in that** the membrane is at least one hollow fibre membrane.

4. Process according to one or more of Claims 1 to 3, **characterised in that** the membrane is hydrophobic.

5. Process according to Claim 4, **characterised in that** the membrane has a microporous structure.

6. Process according to Claim 5, **characterised in that** the membrane has an dense or nanoporous separating layer.

7. Process according to one or more of Claims 1 to 6, **characterised in that** the membrane is made from a polymer based on at least one polyolefin.

8. Process according to Claim 7, **characterised in that** the at least one polyolefin is polypropylene or polymethyl pentene.

9. Process according to one or more of Claims 1 to 3, **characterised in that** the membrane is a silicone membrane or has a silicone coating.

10. Process according to one or more of Claims 1 to 6, **characterised in that** the membrane is made from a polymer based on an aromatic sulfone polymer.

11. Process according to one or more of Claims 1 to 10, **characterised in that** the hyperpolarised gas is xenon.

12. Process according to one or more of Claims 1 to 11, **characterised in that** the liquid in which the hyperpolarised gas is dissolved is a physiological liquid.

13. Process according to Claim 12, **characterised in that** the physiological liquid is blood plasma.

14. Process according to Claim 12, **characterised in that** the physiological liquid is blood.

15. Use of a solution of hyperpolarised gas in a liquid, the solution being produced by a process according to one or more of Claims 1 to 14, as a contrast medium for analytical methods based on magnetic resonance.

16. Use of a solution of hyperpolarised gas in a liquid, the solution being produced by a process according to one or more of Claims 1 to 14, as a contrast medium for analytical methods based on magnetic resonance for examinations of human or animal organs and tissue.

## Revendications

1. Procédé permettant de dissoudre dans un liquide un gaz qui présente des propriétés physiques transitoires, lequel procédé comporte les étapes consistant à prendre un gaz qui présente de propriétés physiques transitoires, à prendre un liquide et à introduire dans ce liquide le gaz qui présente de propriétés physiques transitoires, et lequel procédé est carac-térisé en ce que le gaz qui présente de propriétés physiques transitoires est un gaz hyperpolarisé et en ce que l'on amène ce gaz dans le liquide en lui faisant traverser une membrane semi-perméable qui laisse passer les gaz, laquelle membrane est incorporée dans un boîtier de telle manière que ce boîtier est partagé par la membrane en au moins un espace destiné au liquide et au moins un espace destiné au gaz, ledit espace destiné au liquide étant pourvu d'au moins une entrée et d'au moins une sortie, et ledit espace destiné au gaz étant pourvu d'au moins une entrée pour gaz par laquelle on fait passer le gaz pour l'amener jusqu'à la membrane.

2. Procédé conforme à la revendication 1, **caractérisé en ce que** le gaz est dissous dans le liquide sans qu'il y ait formation de bulles.

3. Procédé conforme à l'une ou plusieurs des revendications 1 et 2, **caractérisé en ce que** la membrane est au moins une membrane à fibres creuses.

4. Procédé conforme à l'une ou plusieurs des revendications 1 à 3, **caractérisé en ce que** la membrane est hydrophobe.

5. Procédé conforme à la revendication 4, **caractérisé en ce que** la membrane présente une structure microporeuse.

6. Procédé conforme à la revendication 5, **caractérisé en ce que** la membrane comporte une couche séparatrice dense ou nanoporeuse.

7. Procédé conforme à l'une ou plusieurs des revendications 1 à 6, **caractérisé en ce que** la membrane est faite en un polymère à base d'au moins une polyoléfine.

8. Procédé conforme à la revendication 7, **caractérisé en ce que** ladite polyoléfine au nombre d'au moins une est un polypropylène ou un poly(méthyl-pentène).

9. Procédé conforme à l'une ou plusieurs des revendications 1 à 3, **caractérisé en ce que** la membrane est une membrane en silicone ou comporte un revêtement de silicone.

10. Procédé conforme à l'une ou plusieurs des revendications 1 à 6, **caractérisé en ce que** la membrane est faite en un polymère à base d'une sulfone aromatique.

11. Procédé conforme à l'une ou plusieurs des revendications 1 à 10, **caractérisé en ce que** le gaz hyperpolarisé est du xénon.

12. Procédé conforme à l'une ou plusieurs des revendications 1 à 11, **caractérisé en ce que** le liquide dans lequel est dissous le gaz hyperpolarisé est un liquide physiologique.

13. Procédé conforme à la revendication 12, **caractérisé en ce que** le liquide physiologique est du plasma sanguin.

14. Procédé conforme à la revendication 12, **caractérisé en ce que** le liquide physiologique est du sang.

15. Utilisation d'une solution d'un gaz hyperpolarisé dans un liquide, préparée suivant un procédé conforme à l'une ou plusieurs des revendications 1 à 14, en tant qu'agent de contraste pour procédés analytiques fondés sur la résonance magnétique.

16. Utilisation d'une solution d'un gaz hyperpolarisé dans un liquide, préparée suivant un procédé conforme à l'une ou plusieurs des revendications 1 à 14, en tant qu'agent de contraste pour procédés analytiques fondés sur la résonance magnétique et servant à l'analyse d'organes et de tissus humains ou animaux.
